# EUROPEAN PATENT APPLICATION

(11) **EP 4 005 542 A1**
(43) Date of publication of application: **01.06.2022**
(21) Application number: 20843381.3
(22) Date of filing: 13.04.2020
(51) Int. Cl.: A61F 9/007, A61M 1/00

(54) **ULTRASONIC SUCTION AND LIQUID INJECTION INTEGRATED DEVICE**

(30) Priority: 23.07.2019 CN 201910666175
(71) Applicant: Innolcon Medical Technology (Suzhou) Co., Ltd., Jiangsu 215000 (CN)
(72) Inventor: YAN, Zhongyu, Jiangsu 215000 (CN); WANG, Hui, Jiangsu 215000 (CN); CHENG, Chunjie, Jiangsu 215000 (CN); LUO, Wei, Jiangsu 215000 (CN)
(74) Representative: Eisenführ Speiser
(86) International application number: PCT/CN2020/084407
(87) International publication number: WO 2021/012719

(57) **Abstract**

This invention discloses an ultrasonic aspiration and irrigation integrated device, comprising a housing (101), a horn (105) placed inside the housing (101), an inner needle tube (201) and an outer needle sleeve (301) extended at the distal end of the housing (101), and the outer needle sleeve (301) is attached to the outside of the inner needle tube (201). The bore inside the inner needle tube (201) is part of an aspiration line, the gap between the inner wall of the outer needle sleeve (301) and the outer wall of the inner needle tube (201) is part of an irrigation line, and at the distal ends of the aspiration line and the irrigation line form a junction area. The invention uses the distal front opening of the inner needle tube (201) to make contact with the irrigation fluid, and the ultrasonic vibration at the inner needle tube (201) distal end produces bubble cavity then explosion, i.e. ultrasonic cavitation, by which to break, liquefy and then aspirate out the target tissue body; the outer sleeve and the provided irrigation liquid effectively enhance the efficiency of tissue liquefaction and extraction, in the meantime minimize the damage of the ultrasonic vibration to the tissue around the needle and reduce the heat generation and its damage.

## Description

### Technical Field

The present invention relates to medical device field, in particular to an ultrasonic aspiration and irrigation device.

### Background

The human eye can form the image by passing through the cornea and focusing through the lens, and then focusing the image on the back retina through the vitreous body. The quality of the focus is determined by a number of factors, including the size and shape of the eye, as well as the transparency of the cornea, lens, vitreous body, etc. The vitreous body is a gel-like transparent tissue located at the posterior segment of the eye, when the eye diseases, such as bleeding in the eye, retina tearing, etc., it needs to be removed by vitrectomy. The removal of the vitreous body can also ease the tension applied to the retina and other tissues of the eye. Commonly used surgical instruments in the vitrectomy include vitrectomy probe (also called vit cutter), light probe, injection tubing, etc.

Such as the Chinese patent CN104640522B " vibration surgical device for the removal of vitreous body and other tissue " published an ultrasonic driven vitrectomy probe, it uses an ultrasonic handpiece to drive the aspiration needle. According to the patent, the device used to remove the vitreous body is due to the periodic two-way flow of tissue through the side port which is caused by the aspirating pressure and the vibration of the needle, while without creating cavities outside the distal end of the needle. For such a device, ultrasonic vibration amplitude is expected to be limited to a very small range to reduce turbulence or tissue damage. In practical applications, a suitable amplitude limit is difficult to control. In addition, ultrasonic vibration and the heat it generated can damage fine tissues such as the retina, without proper cooling and sleeve protection, there will be safety issues for this type of device. Without proper design, the addition of the sleeve will reduce the performance of the ultrasonic vibration and increase heat generating. Although the patent refers to several sheaths attached to the distal end of the needle to reduce damages caused by the turbulence, there is no technical solution to solve the effects to the ultrasonic performance and heating issues, and the aspiration line and irrigation line in the patent are implemented separately by two different instruments.

### Description

In view of this, the present invention provides an ultrasonic aspiration and irrigation integrated device, it uses the distal front opening of the inner needle tube to make contact with the irrigation fluid, and the ultrasonic vibration at the distal end of the inner needle tube produces bubble cavity then explosion, i.e. ultrasonic cavitation, by which to break, liquefy and aspirate the vitreous tissue body, so to reduce the damage of the ultrasonic vibration to the tissue around the needle, to reduce the heat generation and its damage, to provide irrigation liquid to enhance the efficiency of tissue liquefaction and extraction.

In order to solve the above technical problems, the technical scheme of the present invention is:
An ultrasonic irrigation and aspiration integrated device, comprising a housing, a horn placed inside the housing, an inner needle tube and an outer needle sleeve extended at the distal end of the housing, and the outer needle sleeve is attached to the outside of the inner needle tube. The bore inside the inner needle tube is part of an aspiration line, the gap between the inner wall of the outer needle sleeve and the outer wall of the inner needle tube is part of an irrigation line, and at the distal ends of the aspiration line and the irrigation line form a junction area.

Preferably, the irrigation line also includes an irrigation connector and irrigation tube on the housing, and the irrigation tube may be configured as a tube attached to the housing or a channel placed inside the housing.

Preferably, the outer needle sleeve comprises a closed flat distal end, irrigation side port located near the proximal end and aspiration side port located near the distal end.

Preferably, there is a gap between the distal end surface of the inner needle tube and the inner wall of the flat top surface of the outer needle sleeve, and the gap is connected to the aspiration side port.

Preferably, the proximal end of the inner needle tube is attached with a connecting body, and the inner needle tube is fixed on the horn by the connecting body.

Preferably, the connecting body comprises a cone-shaped proximal end with a split, a middle segment with an external thread structure, and a distal end for applying locking force. The outer diameter of the middle segment is greater than the maximum outer diameter of the cone-shaped segment, and the horn has internal thread arranged to match the external thread on the middle segment. The connecting body comprises a central hole, the central hole comprises a large diameter area and a small diameter area; the small diameter area is located in the interior of the cone-shaped proximal end and the middle segment and its diameter is comparable to the outer diameter of the inner needle tube; the large diameter area is located in the interior of distal section and its diameter is greater than the outer diameter of the outer needle sleeve.

Preferably, the large diameter area has a step surface, and the proximal end of the outer needle sleeve is inserted against the step surface.

Preferably, the distal end of the housing is connected with an irrigation soft shoulder sleeve; the distal end of the irrigation soft shoulder sleeve has a needle sleeve holding hole; the needle sleeve holding hole under natural condition its diameter is smaller than the outer diameter of the outer needle sleeve.

Preferably, the irrigation soft shoulder sleeve and the housing are attached with threaded connection.

Preferably, the irrigation soft shoulder sleeve and the housing attached together to form a sealed irrigation space, which forms a part of the irrigation line; one end of the irrigation space through the irrigation side port of the outer needle sleeve is connected to the gap between the inner wall of the outer needle sleeve and the outer wall of the inner needle tube, and the other side is connected to the irrigation connector and the irrigation tube on the housing.

Preferably, the irrigation side port is located within the sealed irrigation space.

The present invention also reveals in more detail another ultrasonic aspiration and irrigation integrated device, comprising a housing, placed inside the housing a horn, extended from the distal end of the housing an inner needle tube and an outer needle sleeve; the outer needle sleeve is attached to outside of the inner needle tube; the proximal end of the inner needle tube is fixed by a connecting body to the distal end of the horn; the proximal end of the outer needle sleeve is attached to the distal end of the connecting body by an irrigation soft shoulder sleeve. The ultrasonic aspiration and irrigation integrated device comprises an aspiration line and an irrigation line; the aspiration line comprises the bore of the inner needle tube, the central hole of the connecting body, the inner hole of the horn and the aspiration connector; the irrigation line comprises a gap between the inner wall of the outer needle sleeve and the outer wall of the inner needle tube, the irrigation space formed by the irrigation soft shoulder sleeve and the housing, the gap between the housing and the horn, the irrigation connector and the irrigation tube; at the distal ends of the aspiration line and the irrigation line form a junction area.

Preferably, the proximal end of the outer needle sleeve and the connecting body are connected with a loose fit.

Preferably, the outer needle sleeve comprises an aspiration side port and an irrigation side port near its two ends respectively; the inlet of the aspiration line is the distal end opening of the inner needle tube, and it is located at the same end of the aspiration side port.

Preferably, the outer needle sleeve comprises a closed flat top surface located at the distal end; there is a gap between the distal end opening of the inner needle tube and the inner wall of the flat top surface of the outer needle sleeve, and the gap is connected with the aspiration side port.

Preferably, the irrigation soft shoulder sleeve is attached with the housing thread and forms a sealed irrigation space, and the irrigation side port is located within the irrigation space.

Preferably, the connecting body comprises a cone-shaped proximal segment with a split, a middle segment with an external thread structure, and a distal segment for applying locking force. The outer diameter of the middle segment is greater than the maximum outer diameter of the cone-shaped segment, and the horn has internal thread arranged to match the external thread on the middle segment. The connecting body comprises a central bore, the central bore comprises a large diameter area and a small diameter area; the small diameter area is located in the interior of the cone-shaped proximal segment and of the middle segment and its diameter is comparable to the outer diameter of the inner needle tube; the large diameter area is located in the interior of the distal segment and its diameter is greater than the outer diameter of the outer needle sleeve. The large diameter area has a step surface, and the proximal end of the outer needle sleeve is inserted against the step surface.

Preferably, the proximal end of the inner needle tube is flush or protruding with the proximal end of the cone-shaped segment, but the protruding length does not exceed one-eighth of the wavelength of the ultrasonic working frequency of the instrument.

Preferably, the proximal section of the inner needle tube is attached to the central bore in the corn-shaped proximal segment of the connecting body by high-strength adhesive.

The beneficial effects of the present invention are mainly reflected on:
1. The aspiration line and the irrigation line formed at the distal end of a junction area, where the internal needle produces the ultrasonic vibration, and because its front opening contacts the irrigation fluid, it is easy to produce cavities, causing cavitation, and then through cavitation to break, liquefy, and aspirate the vitreous body.
2. There is a gap between the outer needle sleeve and inner needle tube, and through the gap flows irrigation liquid; the outer needle sleeve and the connecting body is loose fit, so the ultrasonic vibration on the connecting body and the inner needle tube will not be transmitted to the outer needle sleeve, thus it will not affect the ultrasonic performance of the inner needle tube, but will reduce the ultrasonic vibration caused damage to the tissue around the needle.
3. Ultrasonic vibration itself will cause heat, the elastomer needle sleeve in the prior art will also cause frictional heat, furthermore, vitreous body is viscous and slow moving, will not effectively take away the heat. In the present invention, the aspiration line and the irrigation line form at the distal end of a junction area with flowing fluid through, effectively plays a cooling role, while the irrigation fluid also plays a role of lubricating the aspiration line, reduces the resistance of tube friction, so that improves the aspiration speed.
4. The cavitation produced by the invention plays the role of liquefaction, breaking of viscous vitreous body, making it easier to be aspirated.
5. The internal needle front natural opening is larger than the side opening, so the flow is larger, less resistance, further help to aspirate, improves the efficiency of aspiration.

### Drawings

Fig.1 is a diagram of the ultrasonic aspiration and irrigation integrated device provided by the preferred embodiment of the present invention;
Fig.2 is a partial cross-section view of the ultrasonic aspiration and irrigation integrated device provided by the preferred embodiment of the present invention;
Fig.3 is the exploded diagram of Fig.2;
Fig.4 is a structural diagram of the connecting body provided by the preferred embodiment of the present invention.

### Detailed Description

The following will combine with the specific embodiment shown in the drawings to describe the present invention in detail. However, these embodiments are not limitations to the present invention; the ordinary technical personnel in the art according to these embodiments making the structure, method, or functional transformation are contained in the scope of protection of the present invention.

In the description of the scheme, it should be noted that the terms "center", "up", "down", "left", "right", "front", "back", "vertical", "horizontal", "inside", "outside" and other indications of the orientation or position relationship based on the drawings, only to facilitate the description and simplification of the description, rather than to indicate or imply that the device or component must have a specific orientation, with a specific orientation structure and operation, and therefore cannot be understood as a limitation of the present invention. In addition, the terms "first," "second," and "third" are used only for descriptive purposes and are not understood to indicate or imply relative importance. Also, in the description of the scheme, the operator is used as the reference, the direction close to the operator is the proximal end, and the direction away from the operator is the distal end.

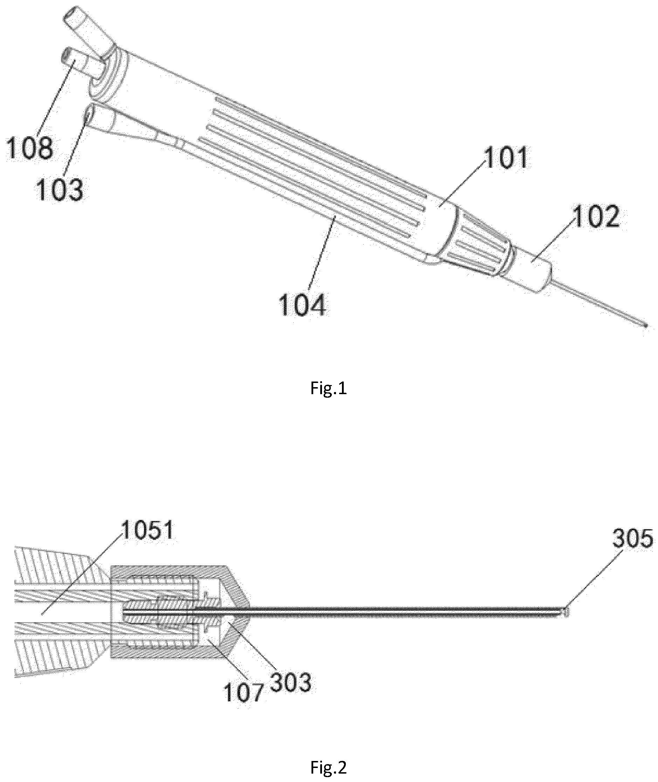

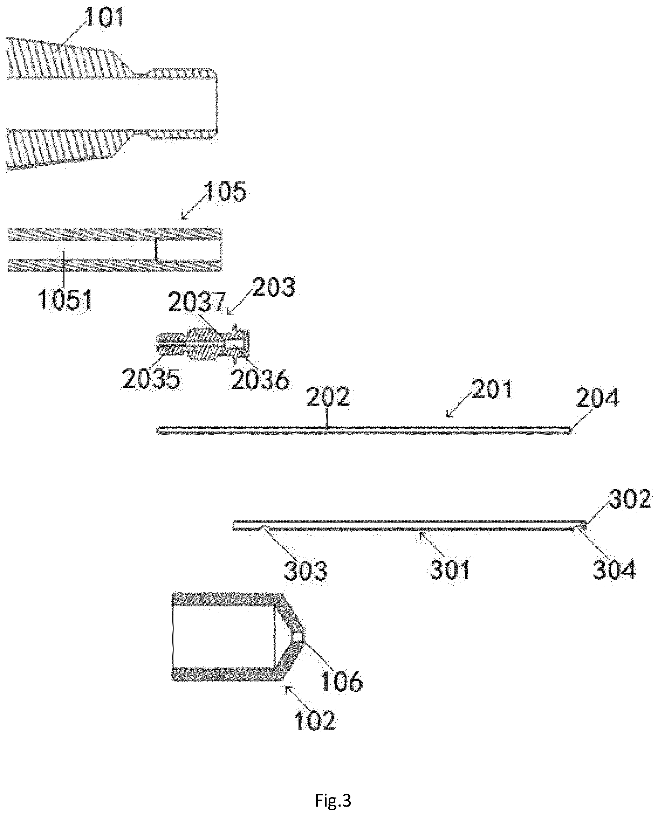

As shown in Figures 1 to 3, the present invention reveals an ultrasonic aspiration and irrigation integrated device, comprising a housing 101, a horn 105 placed inside the housing 101, an inner needle tube 201 and an outer needle sleeve 301 extended at the distal end of the housing 101, and the outer needle sleeve 301 is attached to the outside of the inner needle tube 201.

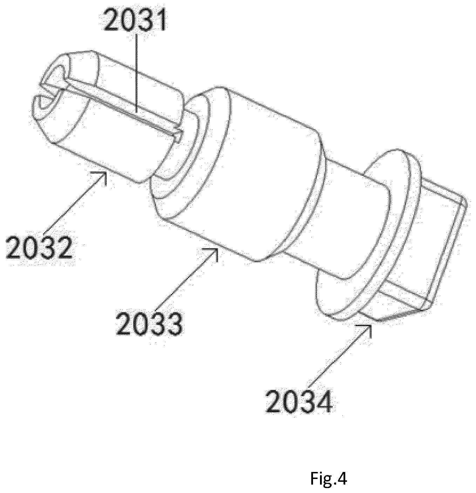

The proximal end of the inner needle tube 201 is fixed at the distal end of the horn 105 by a connecting body 203 (specific structure as shown in Fig.4, detailed later) and the three parts are fixed to each other. The proximal end of the outer needle sleeve 301 is attached at the distal end of the housing 101 by an irrigation soft shoulder sleeve 102. The outer needle sleeve 301 and the connecting body 203 are loose fitted. The specific structure is as following:
The proximal end of the housing 101 is equipped with irrigation connector 103 and aspiration connector 108, and connected respectively, with the irrigation fluid source and the aspiration source producing negative pressure. The irrigation connector 103 and the aspiration connector 108 are respectively used as the proximal end interface of the irrigation line and the aspiration line. The distal end of the housing 101 is attached with an irrigation soft shoulder sleeve 102; the present invention preferably attaches the two by thread connection, and the irrigation soft shoulder sleeve 102 is a tapered structure. The irrigation soft shoulder sleeve 102 has a needle sleeve holding hole 106, the needle sleeve holding hole 106 under natural condition its internal diameter is smaller than the outer diameter of the outer needle sleeve 301, so that when the outer needle sleeve 301 passes through the needle sleeve holding hole 106, the outer needle sleeve 301 is held by the needle sleeve holding hole 106 at a certain position and sealed from leaking.

The horn 105 uses existing technology, its interior includes the inner bore 1051, and the distal end of the inner bore 1051 is an inner threaded structure.

The connecting body 203 comprises a cone-shaped proximal segment 2032 with a split 2031, a middle segment 2033 with an external thread structure, and a distal segment 2034 for applying locking force. The outer diameter of the middle segment 2033 is larger than the maximum outer diameter of the cone-shaped proximal segment 2032, and the outer thread matches the inner thread in the hone 105.

The interior of the connecting body 203 comprises the central bore 2035, the central bore 2035 comprises the large diameter area 2036 and the small diameter area, the small diameter area is located in the interior of the cone-shaped proximal segment 2032 and of the middle segment 2033, and its diameter is comparable to the outer diameter of the inner needle tube 201; the large diameter area 2036 is located in the interior of the distal segment 2034 and its diameter is greater than the outer diameter of the outer needle sleeve 301. The large diameter area 2036 has a step surface 2037, and the proximal end face of the outer needle sleeve 301 is inserted against the step surface 2037, of course, a slight gap is also possible.

When the proximal end of the inner needle tube 201 is extended into the central bore 2035 of the connecting body 203, the proximal end of the inner needle tube 201 is flush or protruding with the proximal end of the cone-shaped proximal segment 2032, but the protruding length does not exceed one eighth of the wavelength of the ultrasonic working frequency of the device, so as not to affect the ultrasonic performance of the system.

In this preferred embodiment, the inner diameter of the small diameter area of the central bore 2035 is slightly larger than the outer diameter of the inner needle tube 201; when the split 2031 tightens, the central bore 2035 in the cone-shaped proximal segment 2032 shrinks, the inner needle tube 201 and the connecting body 203 are locked together. Through this design, the stress of the inner needle tube 201 in the locking section is gradually reduced from the proximal end to the distal direction, so the stress concentration caused by ultrasonic vibration is reduced when working. In order to improve the coupling of the inner needle tube 201 and the connecting body 203, and further reduce the stress concentration, it is recommended to bond the two together in the locking section with high-strength adhesive. When the inner needle tube 201 is inserted in the connecting body 203, the connecting body 203 is screwed into the horn 105 by thread. The length of the thread area inside the horn is shorter than the spin-in length of the connecting body, and the diameter of the inner hole of the horn is less than the outer diameter of the connecting body cone-shaped proximal segment 2032, so that when the connecting body 203 continues to be screwed in, the cone-shaped proximal segment 2032 of the connecting body 203 will hit the inner hole of the horn, chamfer at the contact will compress the split 2031, and finally the inner needle tube 201, the connecting body 203 and the horn 105 are well coupled together.

In this preferred embodiment, the inner needle tube 201 is a linear steel tube, which includes internal bore 202 and distal end opening 204.

The outer needle sleeve 301 is attached to the outside of the inner needle tube 201; the outer needle sleeve 301 comprises an aspiration side port 304 and an irrigation side port 303, respectively, located near both ends. The outer needle sleeve 301 also comprises a closed flat top surface 302 located at the distal end; the distal end opening 204 of the inner needle tube 201 is located on the same end as of the aspiration side port 304, and there is a gap 305 from the inner wall of the flat top surface 302 of the outer needle sleeve 301, the gap 305 is connected with the aspiration side port 304.

The outer needle sleeve 301 inserts from the inner needle tube 201 distal end onto the inner needle tube, and then continues to insert until reachs the step surface 2037 of the large diameter area 2036 in the connecting body 203. The inner diameter of the large diameter area 2036 is greater than the outer diameter of the outer needle sleeve 301, within a range of 50 microns. Thus, the proximal portion of the outer needle sleeve 301 and the connecting body 203 are a gap fit (i.e. loose fit) for controlling the concentricity of the outer needle sleeve 301. Through the needle holding hole 106 at the distal end of the irrigation soft shoulder sleeve 102 the outer needle sleeve 301 is slide in, thus forms a sealed irrigation space 107, and the irrigation side port 303 of the outer needle sleeve 301 is located within the irrigation space 107. One side of the irrigation space 107 through the side port 303 of the outer needle sleeve 301 connects to the gap between the inner wall of the outer needle sleeve 301 and the outer wall of the inner needle tube 201, the other side connects to irrigation connector 103 and the irrigation tube 104 on the housing 101.

As described above, the ultrasonic irrigation and aspiration integrated device forms the aspiration line and irrigation line.

The aspiration line comprises the aspiration side port 304 on the outer needle sleeve 301, the distal end opening 204 of the inner needle tube 201, the internal bore 202 of the inner needle tube 201, the central bore 2035 of the connecting body 203, the inner bore 1051 of the horn 105 and the aspiration connector 108.

The irrigation line comprises a gap between the inner wall of the outer needle sleeve 301 and the outer wall of the inner needle tube 201, the irrigation space 107 formed by the irrigation soft shoulder sleeve 102 and the housing 101, the gap between the housing 101 and the horn 105, the irrigation connector 103, the irrigation tube 104. The irrigation tube 104 may be configured as a tube attached to the housing 101 or a channel placed in the housing 101.

In this preferred embodiment, the inner diameter of the outer needle sleeve 301 is greater than the outer diameter of the inner needle tube 201 in the range of 20-200 microns, so that the gap between is controlled in a certain range to control the speed of irrigation, so that the speed of aspiration is greater than the speed of irrigation, to ensure that the vitreous body can reach to the distal front opening 204 of the inner needle tube 201. Comparing the prior art smaller side opening the distal front opening of the internal needle tube 201 has larger flow, less resistance, thus improves the efficiency of aspiration.

When ultrasonic working, ultrasonic vibration through the transducer and horn 105 propagates to the distal end of the inner needle tube 201; due to the formation of the junction area at the distal end of the aspiration line and irrigation line, the ultrasonic vibration causes cavitation in the junction area to liquefy the vitreous body and together aspirate away with the irrigation fluid.

For the prior art, the principle of aspirating vitreous body is the use of two-way flow, it does not need and try to avoid producing cavitation, because the cavitation will damage the fine tissue of the eye if without proper protection, so it has to carefully control the vibration amplitude of the needle vibration. While in this invention, the principle of aspirating vitreous body is to use cavitation; the presence of non-viscous liquids such as water on the ultrasonic vibration surface, makes it easier to create cavities, causing cavitation. The junction area formed at the distal end of the aspiration line and the irrigation line by this invention is provided with the ultrasonic vibration of the inner needle tube, and because the front opening of the inner needle tube is in contact with the irrigation fluid (e.g. water), the junction area is easy to produce cavities, causing cavitation, and the resulted cavitation plays the role of liquefying, breaking viscous vitreous body, making it easy to be aspirated.

More importantly, in the prior art, the ideal situation is to not cause cavitation, but in many cases it is difficult to control, such as the presence of liquid from another injection needle, ultrasonic-driven aspiration needle amplitude exceeds a certain threshold, etc. The streaming jet and blasting from the cavitation and the vibration of the aspiration needle can all cause damages to eye tissues, so the prior art also proposed to put a protective sleeve on the aspiration needle end, i.e. an elastomer sleeve attached to the aspiration needle. However, the ultrasonic vibration at the needle end is large and sensitive, so it affects ultrasound performance. In this invention, there is a gap between the outer needle sleeve and the ultrasonic driven inner needle tube and through which the irrigation liquid flows, and the loose fit of the connecting body 203 and the irrigation soft shoulder sleeve 102 make the outer needle sleeve 301 center positioned, so the connecting body 203 and the inner needle tube 201 will not transmit ultrasonic vibration to the outer needle sleeve 301, so the outer needle sleeve 301 will not affect the ultrasound performance of the inner needle tube 201; it also reduces the ultrasonic vibration caused damages to the around tissues so to protect fine tissue such as retina.

Ultrasonic vibration itself will cause heat, with the prior art technology, the elastomer sleeve and the aspiration needle vibration will also cause frictional heat, and the vitreous body is very viscous and slow flow, will not effectively take away the heat. In this invention, the junction area formed in the distal end of the aspiration line and the irrigation line has the passage of irrigation fluid, effectively plays a role of cooling, in the meantime the irrigation fluid also plays a role of lubricating the aspiration line, reduces the resistance of line friction, so that the aspiration flow speed is accelerated, efficiency is improved.

The above is only the preferred embodiment of the present invention, it should be pointed out that the above preferred embodiment should not be regarded as a limitation of the present invention. The scope of protection of the present invention shall be defined by the scope of the claims. For ordinary technical personnel in the art, in the essence and scope of the present invention, may also make a number of improvements and modifications, these improvements and modifications should also be regarded as the scope of protection of the present invention.

## Claims

1. An ultrasonic aspiration and irrigation integrated device **characterized by** a housing (101), a horn (105) placed inside the housing, an inner needle tube (201) and an outer needle sleeve (301) extended at the distal end of the housing (101); the outer needle sleeve (301) is attached to the outside of the inner needle tube (201), and the interior bore (202) of the inner needle tube (201) is part of an aspiration line; the gap between the inner wall of the outer needle sleeve (301) and the outer wall of the inner needle tube (201) is part of an irrigation line, and at the distal end of the aspiration line and the irrigation line form a junction area.

2. According to claim 1, the ultrasonic aspiration and irrigation integrated device is **characterized by** that the irrigation line also includes an irrigation connector (103) and an irrigation tube (104) arranged on the housing (101), and the irrigation tube (104) may be configured as a tube attached to the housing (101) or a channel placed in the housing (101).

3. According to claim 1, the ultrasonic aspiration and irrigation integrated device is **characterized by** the outer needle sleeve (301) comprising a closed flat top surface (302) located at the distal end, irrigation side port (303) and aspiration side port (304) located near the two ends of the outer needle sleeve (301) respectively.

4. According to claim 3, the ultrasonic aspiration and irrigation integrated device is **characterized by** a gap (305) between the distal end surface of the inner needle tube (201) and the inner wall of the flat top surface (302) of the outer needle sleeve (301), and the gap (305) is connected with the aspiration side port (304).

5. According to claim 1, the ultrasonic aspiration and irrigation integrated device is **characterized by** the proximal end of the inner needle tube (201) is provided with a connecting body (203), and the inner needle tube (201) is fixed onto the horn (105) by the connecting body (203).

6. According to claim 5, the ultrasonic aspiration and irrigation integrated device is **characterized by** the connecting body (203) comprising a cone-shaped proximal segment (2032) with a split (2031), a middle segment (2033) with an external thread structure, and a distal segment (2034) for applying locking force; the outer diameter of the middle segment (2033) is greater than the maximum outer diameter of the cone-shaped proximal segment (2032), and the horn (105) is provided with a matching internal thread to the external thread on the middle segment (2033); the interior of the connecting body (203) comprises a center bore (2035), the central bore (2035) comprises a large diameter area (2036) and a small diameter area; the small diameter area is located in the interior of the conn-shaped proximal segment (2032) and of the middle segment (2033), and its diameter is similar to the outer diameter of the inner needle tube (201); the large diameter area (2036) is located in the interior of the distal segment (2034) and its diameter is greater than the outer diameter of the outer needle sleeve (301).

7. According to claim 6, the ultrasonic aspiration and irrigation integrated device is **characterized by** that the large diameter area (2036) has a step surface (2037), and the proximal end of the outer needle sleeve (301) is inserted against the step surface (2037).

8. According to claim 1, the ultrasonic aspiration and irrigation integrated device is **characterized by** an irrigation soft shoulder sleeve (102) attached at the distal end of the housing (101); the soft shoulder sleeve (102) at its distal end has a needle sleeve holding hole (106), and the needle sleeve holding hole (106) under natural condition its internal diameter is less than the outer diameter of the outer needle sleeve (301).

9. According to claim 8, the ultrasonic aspiration and irrigation integrated device is **characterized by** a threaded connection between the irrigation soft shoulder sleeve (102) and the housing (101).

10. According to claim 8, the ultrasonic aspiration and irrigation integrated device is **characterized by** the irrigation soft shoulder sleeve (102) and the housing (101)attached together to form a sealed irrigation space (107); the irrigation space (107) formed a part of the injection line; the irrigation space (107) at one side is connected through the irrigation side port (303) to the gap between the inner wall of the outer needle sleeve (301) and the outer wall of the inner needle tube(201), at the other side is connected to the irrigation connector (103) and the irrigation tube (104) on the housing (101).

11. According to claim 10, the ultrasonic aspiration and irrigation integrated device is **characterized by** that the irrigation side port (303) is located within the irrigation space (107).

12. An ultrasonic aspiration and irrigation integrated device **characterized by** a housing (101), a horn (105) placed inside the housing, an inner needle tube (201) and an outer needle sleeve (301) extended at the distal end of the housing (101), the outer needle sleeve (301) is attached to the outside of the inner needle tube (201); the proximal end of the inner needle tube (201) is fixed at the distal end of the horn (105) by a connecting body (203); the proximal end of the outer needle sleeve (301) is attached at the distal end of the housing (101) by an irrigation soft shoulder sleeve (102); the ultrasonic aspiration and irrigation integrated device comprises an aspiration line and an irrigation line; the aspiration line comprises interior bore (202) of the inner needle tube (201), central bore (2035) of the connecting body (203), inner bore of the horn (105) and the aspiration connector (108); the irrigation line comprises the gap between the inner wall of the outer needle sleeve (301) and the outer wall of the inner needle tube (201), the irrigation space (107) between the irrigation soft shoulder sleeve (102) and the housing (101), the gap between the housing (101) and the horn (105), the irrigation connector (103), the irrigation tube (104), the junction area formed by the aspiration line and the irrigation line at the distal end.

13. According to claim 12, the ultrasonic aspiration and irrigation integrated device is **characterized by** a loose fit between the proximal end of the outer needle sleeve (301) and the connecting body (203).

14. According to claim 12, the ultrasonic aspiration and irrigation integrated device is **characterized by** the outer needle sleeve (301) comprising an aspiration side port (304) and an irrigation side port (303) which are arranged at both ends respectively; the inlet of the aspiration line is the distal end opening (204) of the inner needle tube (201), and the distal end opening (204) is located at the same end as the aspiration side port (304).

15. According to claim 14, the ultrasonic aspiration and irrigation integrated device is **characterized by** the outer needle sleeve (301) comprising a closed flat top surface (302) located at the distal end; There is a gap (305) between the end opening (204) of the inner needle tube (201) and the inner wall of the flat top surface (302) of the outer needle sleeve (301), and the gap (305) is connected with the aspiration side port (304).

16. According to claim 14, the ultrasonic aspiration and irrigation integrated device is **characterized by** that the irrigation soft shoulder sleeve (102) is threaded with the housing (101) and forms a sealed irrigation space (107), and the irrigation side port (303) is located within the irrigation space (107).

17. According to claim 12, the ultrasonic aspiration and irrigation integrated device is **characterized by** the connecting body (203) comprising a cone-shaped proximal segment (2032) with a split (2031), a middle segment (2033) with an external threaded structure, a distal segment (2034) for applying locking force; the outer diameter of the middle segment (2033) is greater than the maximum outer diameter of the cone-shaped proximal segment (2032) and the horn (105) is provided with a matching internal thread with the external thread on the middle segment (2033); the interior of the connecting body (203) comprises the central bore (2035), which comprises a large diameter area (2036) and a small diameter area; the small diameter area is located at the interior of the cone-shaped proximal segment (2032) and of the middle segment (2033) and its diameter is comparable to the outer diameter of the inner needle tube (201); the large diameter area (2036) is located in the interior of the distal segment (2034) and its diameter is greater than the outer diameter of the outer needle sleeve (301); the large diameter area (2036) has a step surface (2037), and the proximal end of the outer needle sleeve (301) is inserted against the step surface (2037).

18. According to claim 17, the ultrasonic aspiration and irrigation integrated device is **characterized by** the proximal end of the inner needle tube (201) is flush or protruded with the proximal end of the cone-shaped section (2032), but the protruding length does not exceed one eighth of the wavelength of the ultrasonic operating frequency of the instrument.

19. According to claim 17, the ultrasonic aspiration and irrigation integrated device is **characterized by** the proximal end of the inner needle tube (201) is attached through adhesive to the central bore (2035) of the cone-shaped proximal segment (2032) of the connecting body (203).
